(19) Europäisches Patentamt
European Patent Office
Office européen des brevets

(11) Veröffentlichungsnummer: **0 258 190 B1**

# (12) EUROPÄISCHE PATENTSCHRIFT

(45) Veröffentlichungstag der Patentschrift: **27.11.91**

(21) Anmeldenummer: **87810474.4**

(22) Anmeldetag: **24.08.87**

Die Akte enthält technische Angaben, die nach dem Eingang der Anmeldung eingereicht wurden und die nicht in dieser Patentschrift enthalten sind.

(51) Int. Cl.$^5$: **C07C 41/16**, C07C 319/18, C07C 43/225, C07C 43/29, C07C 321/28, C07C 315/04, C07C 43/205, C07C 43/215, C07C 43/235, C07C 205/35, C07C 217/14

## (54) Verfahren zur Herstellung von aromatischen Ether- und Thioetherverbindungen.

(30) Priorität: **29.08.86 CH 3480/86**

(43) Veröffentlichungstag der Anmeldung: **02.03.88 Patentblatt 88/09**

(45) Bekanntmachung des Hinweises auf die Patenterteilung: **27.11.91 Patentblatt 91/48**

(84) Benannte Vertragsstaaten: **BE CH DE FR GB IT LI NL**

(56) Entgegenhaltungen:
**EP-A- 0 052 314**
**DE-A- 2 805 983**

**PATENT ABSTRACTS OF JAPAN, Band 9, Nr. 96 (C-278) (1819), 25. April 1985 & JP-A-59 227836**

**METHODEN DER ORGANISCHEN CHEMIE HOUBEN-WEYL, Band 6/3, Seiten 54, 55, Georg Thieme Verlag, Stuttgart, 1965; EUGEN MUELLER et al; & THE JOURNAL OF THE AMERICAN CHEMICAL SOCIETY, Band 55, Seiten 3718-3721, 1933**

**ROEMPPS CHEMIE-LEXIKON, 8. Auflage, Franckh'sche Verlagshandlung, Stuttgart, Seite 4240**

(73) Patentinhaber: **CIBA-GEIGY AG**
**Klybeckstrasse 141**
**CH-4002 Basel(CH)**

(72) Erfinder: **Frey, Christian, Dr.**
**Rebgasse 38**
**CH-4132 Muttenz(CH)**
Erfinder: **Dill, Bernd, Dr.**
**Brunnenweg 19**
**W-7888 Rheinfelden 4(DE)**

**EP 0 258 190 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein neues Verfahren zur Herstellung von aromatischen Ether- und Thioether-Verbindungen.

Gemäss JP-A-59-22 78 36 können diese aus einem Phenol bzw. Thiophenol in Gegenwart eines Carbonats und Methylisobutylketon sowie einem Alkylhalogenid unter Rückflussbedingungen erhalten werden. Bei der Nacharbeitung dieses Verfahrens stellt man jedoch fest, dass der Umsatz unter den gennanten Bedingungen zu wünschen lässt. Insbesondere sei die Reaktion von Ethylbromid mit 3-Diethylaminophenol gemäss JP-A-58-227836 erwähnt wo relativ viel Ausgangsmaterial zurückerhalten wird. Das gennante Verfahren ist demnach offenbar nicht ohne weiteres auf alle in Frage kommenden Alkylierungsmittel übertragbar.

Es wurde nun gefunden, dass die Anwendungsbreite des im JP-A-59-227836 beschriebenen Verfahrens wesentlich vergrössert werden kann, wenn man die Reaktion bei Temperaturen von 115 bis 200°C, bevorzugt bei 120 bis 150°C, durchführt und dabei unter Druck arbeitet. Dieses Vorgehen macht einerseits Gebrauch von der günstigen druckabhängigen Löslichkeit selbst von niedermolekularen, leicht flüchtigen Alkylhalogeniden wie z.B. Ethylchlorid oder Methylbromid in der flüssigen Ketonphase, andererseits von der druckbedingten Gleichgewichtsverschiebung des Systems Alkalicarbonat-Wasser-$CO_2$-Hydrogencarbonat in Richtung des Hydrogencarbonates. Indem weder Wasser noch $CO_2$ aus dem System ausgeschleust werden muss, gibt es keinen Verlust von dampfförmig entweichendem Alkylhalogenid, was sowhol oekologisch als auch oekonomisch von hoher Bedeutung für eine umweltgerechte Fabrikation ist. Durch Vermeidung von Rückflusstemperaturen kann so auch erheblich Energie eingespart werden. Ueberraschenderweise gelingt es trotz Anwendung von Temperaturen, die über dem Siedepunkt des Lösungsmittels und zum Teil weit über jenem der Alkylhalogenide liegen können, die Summe der Partialdrücke von $CO_2$, Lösungsmittel und Alkylhalogenid in der Gasphase auf den Bereich von 0,5 bis 10 bar Ueberdruck zu begrenzen. Dies ist von grosser technischer und oekonomischer Bedeutung, weil statt teurer Autoklaven normale, druckbelastbare Rührkessel eingesetzt werden können. Ein weiterer Vorteil dieser Verfahrensweise ist der Wegfall von Dosiervorrichtungen zur Zugabe des Alkylhalogenids. Der Reaktor kann bei Raumtemperatur geladen werden.

Es wurde weiter gefunden, dass sich das erfindungsgemässe Verfahren nicht nur auf Alkylhalogenide beschränkt; es ist auch auf Arylhalogenide - insbesondere solche der Anthrachinonreihe -anwendbar. Als Beispiel sei die Herstellung von 1,8-Dihydroxy-anthrachinon aus 1,8-Dichlor-anthrachinon und Phenol genannt.

Die vorliegende Erfindung betrifft somit ein Verfahren zur Herstellung von aromatischen Ether- und Thioetherverbindungen der Formel I

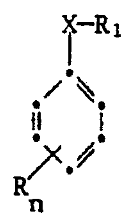

(I)

durch Umsetzung von Verbindungen der Formel II

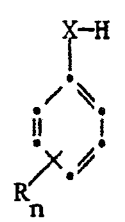

(II)

mit Verbindungen der Formel III

$R_1$ - Y (III)

worin bedeuten:

- R einen Substituenten,
- n die Zahlen 0, 1 oder 2,
- X Sauerstoff, Schwefel oder $-SO_2-$,
- $R_1$ gegebenenfalls substituiertes Alkyl, gegebenenfalls substituiertes Alkenyl oder Aryl (z.B. Phenyl, Naphthyl, Anthrachinoyl), und
- Y ein Halogenatom (z.B. Chlor, Brom, Iod), wobei dieses Y ein- oder mehrmals vorhanden sein kann,

in Gegenwart eines Säureakzeptors in einem aliphatischen Keton als Lösungsmittel, indem man die Reaktion unter Druck bei Temperaturen von 115 bis 200 °C ausführt.

Die Ether- und Thioetherverbindungen der Formel (I) stellen wertvolle Zwischenprodukte vor allem für basische Farbstoffe dar.

Der bevorzugte Temperaturbereich liegt zwischen 120 und 150, insbesondere 140 und 145 °C; der Druck, der zur Anwendung kommt, liegt zwischen 0,5 und 10,0 bar bevorzugt zwischen 0,5 und 2,0 bar und ist abhängig vom verwendeten aliphatischen Keton und vom Alkylierungsmittel der Formel (III).

Als Säureakzeptoren werden vor allem anorganische basische Salze verwendet wie z.B. die Oxide, Hydroxyde, Hydrogencarbonate und Carbonate der Alkali- und Erdalkalimetalle. Es kommen beispielsweise in Frage: Lithiumhydroxyd, Natriumhydroxyd, Kaliumhydroxyd, Calciumhydroxyd, Bariumhydroxyd, Magnesiumoxid, Lithiumhydrogencarbonat, Natriumhydrogencarbonat, Kaliumhydrogencarbonat, Calciumhydrogencarbonat, Bariumhydrogencarbonat, Lithiumcarbonat, Natriumcarbonat, Kaliumcarbonat, Calciumcarbonat und Bariumcarbonat.

Als Lösungsmittel kommt ein aliphatisches Keton oder eine Mischung derartiger Ketone zur Anwendung. Es handelt sich dabei vor allem um solche der Formel IV

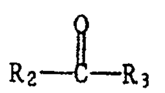

(IV)

worin $R_2$ und $R_3$ unabhängig voneinander $C_1$-$C_4$-Alkyl bedeuten. Genannt sind in diesem Zusammenhang z.B. Methyl-ethyl-keton, Diisopropylketon und Methyl-isobutyl-keton.

R in der Verbindung der Formel (I) ist ein beliebiger Substituent, der den erfindungsgemässen Reaktionsablauf nicht beeinträchtigt, so z.B. Halogen (z.B. Fluor, Chlor, Brom); Carboxyl; Alkoxycarbonyl wie Methoxycarbonyl, Aethoxycarbonyl, Allyloxycarbonyl und Alkenyloxycarbonyl; $C_1$-$C_4$-Alkyl; heterocyclish substituiertes Alkyl wie Pyrrolidin substituiertes Methyl und Propyl; Acyl wie Acetyl; $NO_2$; Dialkyl($C_1$-$C_4$)amino wie Dimethylamino und Diethylamino

Als Substituenten $R_1$ kommen die gleichen in Frage wie oben unter R definiert. Unter Alkyl($C_1$-$C_6$) ist z.B. zu verstehen Methyl, Ethyl, Propyl, Isopropyl, Butyl, Isobutyl, Pentyl, Isopentyl, Hexyl. Als Allyl($C_1$-$C_6$) ist beispielsweise genannt: 2-Allyl, 2-Butenyl, 3-Butenyl und 4-Butenyl.

Kommt Y mehrmals in der Verbindung (III) vor, so versteht es sich, dass im Endprodukt der Verbindung (I) auch mehrmals der Rest der Verbindung (II) der Formel

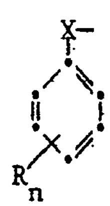

vorkommen kann.

Ein weiterer Vorteil des erfindungsgemässen Verfahrens liegt darin, dass die nach dem Verfahren entstandenen Halogenide der Alkali- oder Erdalkalimetalle durch einfaches Abfiltrieren oder Auswaschen abgetrennt werden können. Insbesondere wird hervorgehoben, dass Alkalibromide (beispielsweise Kaliumbromid) auf diese Weise zur Regenerierung von Brom in fester Form abgeschieden werden können. Die Abgabe von Bromidionen ins Abwasser wird dadurch vermieden. Die folgenden Beispiele veranschaulichen die Erfindung.

Beispiel 1:

In einem 1 ℓ-Reaktor werden 300 ml Methyl-isobutyl-keton vorgelegt, sodann 73.1 g 3-Diethylamino-phenol und 153.3 g gemahlenes Kaliumcarbonat eingetragen und 30 Minuten verrührt. Anschliessend werden nooh 72.7 g Ethylbromid in den Autoklaven gegeben. Der Autoklav wird sodann verschlossen, während 3 Stunden auf 115 - 120° C erhitzt und dann weitere 5 Stunden bei 140 - 142° C gehalten (Druck: 5 bar). Anschliessend lässt man auf ca. 25° C kühlen und trennt das Reaktionsprodukt der Formel

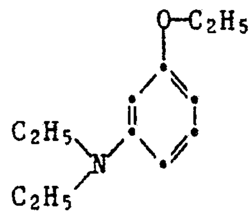

nach Zugabe von Wasser mit der organischen Phase ab und wäscht diese mit Wasser aus. Nach dem Eindampfen des Lösungsmittels erhält man 80,9 g (Ausbeute: 97,5 %) eines 96,3 %igen Produktes.

Beispiel 2:

In einem 1 ℓ-Reaktor werden 300 ml Methyl-isobutyl-keton, 73,1 g 3-Diethylamino-phenol und 153,3 g gemahlenes Kaliumcarbonat vorgelegt und 30 Minuten verrührt. Anschliessend werden noch 72,7 g Ethylbromid in den Autoklaven gegeben. Der Autoklav wird sodann verschlossen und innert 3 Stunden auf 115 - 120° C erhitzt. Dann wird weitere 5 Stunden bei 140 - 142° C gehalten (Druck: 2,7 bar). Anschliessend lässt man auf ca. 25° C kühlen und filtriert vom gebildeten Kaliumbromid ab. Man wäscht mit wenig Methyl-isobutyl-keton nach und erhält nach dem Eindampfen der vereinigten Filtrate 75,6 g 3-Diethylamino-phenetol (91,1 % Ausbeute) mit einem Titer von 96,9 %.

Beispiel 3:

In einem 1 ℓ-Reaktor werden 150 ml Methyl-isobutyl-keton, 73,1 g 3-Diethylamino-phenol und 153,3 g gemahlenes Kaliumcarbonat vorgelegt. Nach 30-minütigem Verrühren werden noch 72,7 g Ethylbromid beigefügt und der Autoklav wird verschlossen. Es wird während 3 Stunden zunächst auf 115 - 120° C, dann während weiterer 5 Stunden auf 140 - 142° C erhitzt (Druck: 4 bar). Nach dem Kühlen auf ca. 25° C und Versetzen mit Wasser wird das Produkt mit der organischen Phase abgetrennt. Diese wird mit Wasser nachgewaschen und eingedampft. Man erhält 80,67 g 3-Diethylamino-phenetol (97,2 % Ausbeute ) mit einem Titer von 94,8 %.

Beispiel 4:

In einem 1 ℓ-Reaktor werden 300 ml Methyl-isobutyl-keton, 79,17 g 2,4-Dinitro-phenol 100 % und 175,9 g gemahlenes Kaliumcarbonat vorgelegt und 63,6 g Methylbromid zugefügt. Der Autoklav wird verschlossen und innert 3 Stunden auf 115 - 120° C erhitzt (Druck: 3,5 barü). Dann wird während weiteren 5 Stunden noch bei 140 - 142° C gehalten (Druck: 5 - 3 barü, fallend). Nach dem Kühlen auf ca. 25° C wird das Lösungsmittel bei 50° C im Teilvakuum abdestilliert und der salzhaltige Rückstand in warmem Wasser aufgenommen. Nach dem Abfiltrieren des gebildeten 2,4-Dinitro-anisols wird noch mit Ammoniaklösung 1 % und mit Warmwasser (ca. 50° C) nachgewaschen. Nach dem Trocknen im Vakuum bei 50° C wurden 84,6 g 2,4-Dinitro-anisol mit einem Gehalt von 91,8 % erhalten (91,2 % Ausbeute).

Beispiel 5:

In einem druckbelastbaren Rührkessel werden 183 ml Di-i-butylketon vorgelegt. Unter Rühren trägt man 86,3 g Phenol (90 %) ein. Während 5 Minuten werden 103,8 g 1,8-Dichloranthrachinon eingetragen und die Reaktionsmischung zum Kochpunkt erhitzt. Bei 155° C beginnt Wasser abzudestillieren. Nach 1,5 Stunden Reaktionszeit bei dieser Temperatur lässt man auf 35° C abkühlen und gibt dann 67 g Kaliumcarbonat zu. Nun wird während 90 Minuten auf 160° C erwärmt und man lässt 1 Stunde bei dieser Temperatur

reagieren. Abdestillierendes Wasser wird entfernt. Der Rührkessel wird verschlossen, auf 175 bis 176° C geheizt und 5 Stunden bei dieser Temperatur gehalten. Nach Abkühlen auf 30° C wird der Rührkessel entspannt. Das Lösungsmittel wird destillativ entfernt. Der Reaktionsmischung setzt man 3 ml 50 %ige Natriumhydroxidlösung zu, filtriert das 1,8 Diphenoxy-anthrachinon und trocknet es im Vakuum. Man erhält so 141,0 g Produkt, mit einem Gehalt von 86,2 % was einer Ausbeute von 89,0 % entspricht. Der Umsatz beträgt 99,9 %.

Beispiel 6

224,4 g 3-Diethylaminophenol, 64,4 g Natriumhydroxid und 20,4 g Natriumcarbonat werden in einem druckbelastbaren Rührkessel in 340 g Methyl-i-butylketon vorgelegt und dieser verschlossen. Nach Einpressen von 134,4 g Ethylchlorid wird während 3-Stunden auf 100° C, weiteren 4 Stunden auf 115° C und einer weiteren Stunde auf 140° C erhitzt. Man hält die Reaktionsmischung bei dieser Temperatur während 10 Stunden. Es stellt sich ein Enddruck von etwa 5 barü ein. Nach Abkühlen auf 25° C wird der Rührkessel entspannt. Der Kesselinhalt wird mit Wasser ausgespült und die organische Phase wird dann von der wässrigen getrennt. Aus der organischen Phase können nach Entfernen des Lösungsmittels 268 g 3-Diethylaminophenetol isoliert werden, was eine Ausbeute von 97,1 % und einem Umsatz von 99,8 % entspricht.

Beispiel 7:

360 Teile Methyl-isobutylketon und 132,7 Teile 4-Chlorbenzol-sulfinsäure Na-Salz à 100 % (0,665 Mol; als freie Säure mit M. 176,6 gerechnet) werden vorgelegt, unter Rühren zum Sieden erhitzt und das Wasser über einen Abscheider azeotrop abgetrennt. Anschliessend wird auf ca. 25° C abgekühlt. Sodann wird diese Suspension mit 115,9 Teilen gemahlenem Kaliumcarbonat (0,83 Mol) und 108,7 Teilen Ethylbromid (0,998 Mol) versetzt.

Dieses Gemisch wird sodann in verschlossenem Gefäss in 3 Std. auf 115-120° C, und dann in 1 Std. auf 140-142° C erhitzt. Man hält noch weitere 5 Std. bei 140-142° C (Druck: 3 bar) und kühlt dann auf 25° C ab. Die Reaktionsmasse wird dann mit 200 Teilen Wasser von 50° C versetzt, durchgerührt und abtrennen gelassen.

Nach dem Eindampfen der organischen Phase erhält man 95 Teile 4-Chlor-phenyl-ethylsulfon (89,3 % der Theorie).

**Patentansprüche**

**1.** Verfahren zur Herstellung von aromatischen Ether-, Thioetherverbindungen und Sulfonen der Formel I

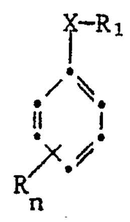

(I)

durch Umsetzung von Verbindungen der Formel

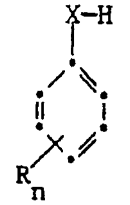

(II)

mit Verbindungen der Formel

$R_1$ - Y (III)

worin bedeuten:

- R einen Substituenten,
- n die Zahlen 0, 1 oder 2,
- X Sauerstoff, Schwefel oder $-SO_2-$,
- $R_1$ gegebenenfalls substituiertes Alkyl, gegebenenfalls substituiertes Alkenyl oder Aryl und
- Y ein Halogenatom

in Gegenwart eines Säureakzeptors in einem aliphatischen Keton als Lösungsmittel, dadurch gekennzeichnet, dass die Reaktion unter Druck zwischen 0,5 bis 10,0 bar, bei Temperaturen von 115 bis 200 °C ausgeführt wird.

2. Verfahren nach Anspruch 1, dadurch gekennzeichnet, dass die Temperatur 120 bis 150 °C beträgt.

3. Verfahren gemäss Anspruch 1, dadurch gekennzeichnet, dass als aliphatisches Keton ein solches der Formel

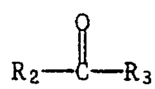

(IV)

verwendet wird, worin $R_2$ und $R_3$ unabhängig voneinander $C_1$-$C_4$-Alkyl bedeuten.

4. Verfahren gemäss Anspruch 3 dadurch gekennzeichnet, dass als aliphatisches Keton Methyl-ethylketon, Diisopropylketon oder Methyl-isobutyl-keton verwendet wird.

5. Verfahren gemäss Anspruch 1 dadurch gekennzeichnet, dass die Reaktion bei einer Temperatur von 140 bis 145 °C durchgeführt wird.

6. Verfahren gemäss Anspruch 1 dadurch gekennzeichnet, dass als Säureakzeptoren anorganische basische Salze verwendet werden.

7. Verfahren gemäss Anspruch 6, dadurch gekennzeichnet, dass als anorganische basische Salze die Hydroxyde, Hydrogencarbonate und Carbonate der Alkali- und Erdalkalimetalle verwendet werden.

**Claims**

1. A process for the preparation of an aromatic ether or thioether compound or of a sulfone of formula I

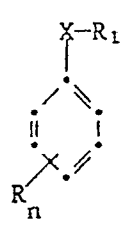

(I)

by reacting a compound of formula

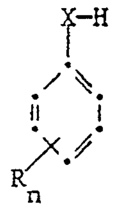

(II)

with a compound of formula

$R_1$ - Y (III)

in which

- R is a substituent,
- n is the number 0, 1 or 2,
- X is oxygen, sulfur or $-SO_2-$,
- $R_1$ is substituted or unsubstituted alkyl, substituted or unsubstituted alkenyl, or aryl, and
- Y is a halogen atom,

in the presence of an acid acceptor and in an aliphatic ketone as solvent, which process comprises carrying out the reaction under pressure at from 0.5 to 10 bar in the temperature range from 115 to 200 °C.

2. A process according to claim 1, wherein the temperature is in the range from 120 to 150 °C.

3. A process according to claim 1, wherein the aliphatic ketone used is one of formula

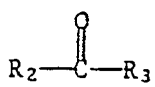

(IV)

in which $R_2$ and $R_3$ are each independently of the other $C_1$-$C_4$alkyl.

4. A process according to claim 3, wherein the aliphatic ketone used is methyl ethyl ketone, diisopropyl ketone or methyl isobutyl ketone.

5. A process according to claim 1, wherein the reaction is carried out in the temperature range from 140 to 145 °C.

6. A process according to claim 1, wherein the acid acceptor used is an inorganic base salt.

7. A process according to claim 6, wherein the inorganic base salt used is a hydroxide, bicarbonate or carbonate of an alkali metal or alkaline earth metal.

**Revendications**

1. Procédé de préparation d'éthers, thioéthers et sulfones aromatiques de formule I

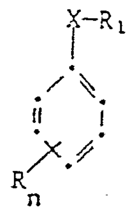

(I)

par réaction de composés de formule

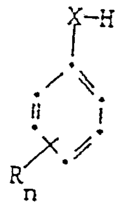

(II)

avec des composés de formule

$R_1$ - Y (III)

dans lesquelles formules :

- R représente un substituant,
- n représente le nombre 0, 1 ou 2
- X représente un atome d'oxygène ou de soufre ou un groupe $-SO_2-$,
- $R_1$ représente un groupe alkyle éventuellement substitué, alcényle éventuellement substitué ou aryle, et
- Y représente un atome d'halogène,

en présence d'un accepteur d'acide, dans une cétone aliphatique en guise de solvant, le procédé étant **caractérisé** en ce que l'on effectue la réaction sous une pression valant entre 0,5 et 10 bars et à des températures de 115 à 200 °C.

2. Procédé conforme à la revendication 1,
**caractérisé** en ce que la température vaut de 120 à 150 °C.

3. Procédé conforme à la revendication 1,
**caractérisé** en ce que l'on utilise, en tant que cétone aliphatique, une cétone de formule

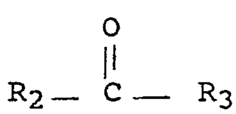

(IV)

dans laquelle $R_2$ et $R_3$ représente, indépendamment l'un de l'autre, des groupes alkyle en $C_{1-4}$.

4. Procédé conforme à la revendication 3,
**caractérisé** en ce que l'on utilise, comme cétone aliphatique, la méthyl-éthyl-cétone, la di-isopropyl-cétone ou la méthyl-isobutyl-cétone.

5. Procédé conforme à la revendication 1,
**caractérisé** en ce que l'on effectue la réaction à une température de 140 à 145 °C.

6. Procédé conforme à la revendication 1,
**caractérisé** en ce que l'on utilise, comme accepteurs d'acide, des sels minéraux basiques.

7. Procédé conforme à la revendication 6, **caractérisé** en ce que l'on utilise, comme sels minéraux basiques, les hydroxydes, hydrogénocarbonates et carbonates de métaux alcalins ou alcalino-terreux.